# EUROPEAN PATENT APPLICATION

(11) **EP 4 070 824 A1**
(43) Date of publication of application: **12.10.2022**
(21) Application number: 21167738.0
(22) Date of filing: 09.04.2021
(51) Int. Cl.: A61L 27/36, A61L 27/38, B33Y 70/00

(54) **MARINE SCAFFOLDS FOR HUMAN TISSUE GENERATION**

(71) Applicant: Regen i Göteborg AB, 413 25 Göteborg (SE)
(72) Inventor: Sumitran, Suchitra, 41325 Göteborg (SE); Holgersson, Vanessa, 41325 Göteborg (SE)
(74) Representative: AWA Sweden AB

(57) **Abstract**

The present invention relates to a method for generation of personalized tissue-engineered human grafts. The method comprising the following steps in the order named: a) providing a body part taken from a marine invertebrate, the body part comprising at least two biologically interconnected tissues selected from the group consisting of connective tissue, muscle tissue, epithelia tissue, and nervous system tissue; b) washing the body part with distilled water; c) decellularizing the washed body part to remove cells and DNA, to provide a decellularized 3D-scaffold with extracellular matrix (ECM) proteins, and, optionally, d) recellularizing the decellularized 3D-scaffold by: inoculating human stem cells into the decellularized 3D-scaffold, and in vitro culturing the human stem cells in the inoculated 3D-scaffold.

## Description

### FIELD OF THE INVENTION

The present inventive concept relates to the field of marine scaffold matrices for generation of personalized tissues for future clinical application. More particularly, the inventive concept relates to a method for generation and evaluation of personalized human grafts using decellularized marine invertebrates as novel bio-scaffolds recellularized with patient's autologous stem cells.

### BACKGROUND

Osteoarthritis (OA) is a degenerative joint disease that affects millions of people worldwide. The disease is characterized by progressive loss of hyaline cartilage in the synovial joints which leads to significant joint pain, swelling and stiffness for sufferers. The disease is also a significant economic burden. The current gold standard treatment option for OA is total joint arthroplasty where the diseased cartilage and underlying bone are replaced with a metal and polymer prosthesis. While the procedure is well established failures and complications are not uncommon. For tissues such as cartilage the need for new approaches is large since the body's own capability to produce and heal cartilaginous tissue is poor.

Bone is well known for its self-healing abilities. Nevertheless, in pathological fractures or large and massive bone defects, bone healing and repair fail. Major bone reconstruction procedures often use autografts, allografts, or xenografts to improve bone healing, but these have their disadvantages and limitations.

Advances in tissue engineering have facilitated the development of replacement tissues or organs for the treatment of injured or degenerative tissue. Scaffolds represent important components for tissue engineering. One group of biological scaffold materials that are already commonly in use for a variety of reconstructive surgical applications is that derived from the extracellular matrix (ECM). Such scaffolds are produced by the process of decellularization of tissue/organs.

The rationale behind using native matrix materials is the isolation of ECM proteins that are site-specific and provide protein 'footprints' of previous resident cells. ECM derived from decellularization of various organs and tissues such as liver, bladder, blood vessel, lung, bone, etc has shown promising results in creating a biological scaffold capable of providing a favourable environment for survival, proliferation and differentiation of the resident cells (stem cells/primary cells).

WO2012/058617 discloses a method for forming a bone tissue module comprising inducing different progenitor cells to form osteogenic progenitor cells, expanding the osteogenic progenitor cells, combining the osteogenic progenitor cells and a scaffold, i.e. decellularized bone and incubating the osteogenic progenitor cells and the scaffold, seeding the connective tissue particulates with cultivated cells under such conditions that the cells adhere to the connective tissue particulates.

WO94/03584 discloses a method of producing graft tissue, which comprises the steps of: a) freezing a connective tissue source having living cells i.e. a marine animal connective tissues or produced from extracellular matrix proteins; b) processing the connective tissue source to remove the cell remnants from the connective tissue source without removing factors necessary for cell growth to form a processed connective tissue source; and c) fragmenting the processed connective tissue source to produce connective tissue particulates, thereby producing graft tissue, seeding the connective tissue particulates with cultivated cells under such conditions that the cells adhere to the connective tissue particulates. However, the product obtained by this method is suitable for 3D-printing but is less suitable for direct use since the connective tissue is fragmented into particulates.

So far, research has been focused mainly on decellularizing mammalian scaffolds ignoring diverse opportunities like regenerative ability of marine invertebrates. Marine life and its rich biodiversity provide a plentiful resource of potential new products for the society. Remarkably, marine organisms remain a largely unexploited resource for biotechnology applications. A possible sustainable source of scaffolds could be marine invertebrates, more precisely species with high regenerative capabilities such as starfish. Moreover, these do not have a central nervous system which is why their use does not raise many ethical issues.

CN110193096 discloses a marine-derived biomimetic cartilage material and a preparation method thereof. The marine-derived cartilage may derive from fish cartilage, shark cartilage, stingray cartilage, mackerel cartilage, squid cartilage, etc. The method comprises the steps of decellularizing the cartilage for direct use in human bodies. However, the method specifically makes use of cartilage which means the cartilage must be isolated from the remaining part of the marine subject prior to decellularization. Another problem lies in that the product obtained is not adapted for humans and therefore it takes time for the human body to accept it as graft since no human cells are introduced.

Thus, there remains need for improved methods for generation of personalized tissue-engineered human grafts for direct use.

### SUMMARY

An object of the disclosure is to provide a method for generation of personalized tissue-engineered human grafts for direct use.

According to a first aspect of the disclosure, these and other objects are achieved, in full or at least in part, by a method for generation of personalized tissue-engineered human grafts, the method comprising the following steps in the order named:
a) providing a body part taken from a marine invertebrate, the body part comprising at least two biologically interconnected tissues selected from the group consisting of connective tissue, muscle tissue, epithelia tissue, and nervous system tissue;
b) washing the body part with distilled water;
c) decellularizing the washed body part to remove cells and DNA, to provide a decellularized 3D-scaffold with extracellular matrix (ECM) proteins, and, optionally,
d) recellularizing the decellularized 3D-scaffold by:
   inoculating human stem cells into the decellularized 3D-scaffold, and
   in vitro culturing the human stem cells in the inoculated 3D-scaffold.

The method provides the advantage that the starting material comprises tissue that is maintained in its natural environment, i.e. the tissues are not separated from each other. Some advantages of using unseparated tissues are presence and accessibility of multitude nutrients which enhances tissue growth. Furthermore, time for separation processes is saved and valuable materia is not cut off. Moreover, it is an advantage to use whole body parts of marine invertebrates since it better resembles human tissue and therefore suits for generation of tissue engineered human grafts for direct use.

According to one example embodiment, a method for generation of personalized tissue-engineered human grafts for direct use, the method comprising the following steps in the order named:
a) providing a body part taken from a marine invertebrate, the body part comprising at least two biologically interconnected tissues selected from the group consisting of connective tissue, muscle tissue, epithelia tissue, and nervous system tissue;
b) washing the body part with distilled water;
c) decellularizing the washed body part to remove cells and DNA, to provide a decellularized 3D-scaffold with extracellular matrix (ECM) proteins, and,
d) recellularizing the decellularized 3D-scaffold by:
   inoculating human stem cells into said decellularized 3D-scaffold, and
   in vitro culturing said human stem cells in said inoculated 3D-scaffold.

### Marine invertebrates

Many marine invertebrates have capacity to regenerate their internal and exterior organs and thus constitute suitable scaffolds for tissue engineering. Furthermore, many marine invertebrates possess ECM proteins that are immunologically related to known ECM proteins found in vertebrates, e.g. fibronectin, laminin, fibrillar collagen which has strong similarities with collagen type I, and glycosaminoglycans which belongs to the sulphate family. They also may possess a hyalin layer, an apical lamina, and the basal lamina which is beneficial since it provides a more complex and robust structure. Furthermore, several matrix metalloproteinases and tissue inhibitors of metalloproteinases have been found in marine invertebrates. Marine invertebrates thus constitute a prominent and sustainable source for scaffolds for tissue engineering.

According to one example embodiment, the body part derive from Echinoderms, Poriferans, Cnidarians, Mollusks, or Arthropods.

Non-limiting examples of poriferans are sponges, of Cnidarians are sea Jellies and Corals, of Mollusks are octopuses, snails, and clams, and of Arthropods are insects, spiders, and lobsters.

According to one example embodiment, the body part derive from echinoderms such as starfish, sea urchins, sand dollars, sea cucumbers, and sea lilies.

According to one example embodiment, the body part derive from Echinoderms, Poriferans, Cnidarians, Mollusks, or Arthropods; or wherein the body part derive from Echinoderms such as starfish, sea urchins, sand dollars, sea cucumbers, or sea lilies.

### Decalcification

According to one example embodiment, the method further comprises a step of decalcifying the washed body part of step b) before the step of decellularizing. Decalcification describes a technique for removing calcium ions and/or other mineral from bone or other calcified tissues in order to soften. Decalcification is thus advantageous in cases of bony and/or hard tissue structures.

In one example embodiment, the step of decalcification is employed by a decalcification media comprising an acid, such as hydrochloric acid, nitric acid, and formic acid.

In one example embodiment, the step of decalcification is employed by a decalcification media comprising an acid and a chelating agent.

In one example embodiment, the step of decalcification is employed by a decalcification media comprising a chelating agent. Non-limiting examples of chelating agents are ethylenediaminetetraacetic acid (EDTA), nitrilotriacetic acid (NTA), iminodisuccinic acid (IDS), polyaspartic acid, S,S-ethylenediamine-N,N'-disuccinic acid (EDDS), methylglycinediacetic acid (MGDA), L-Glutamic acid N,N-diacetic acid, and tetrasodium salt (GLDA).

In one example embodiment, the tissue is shaken during the step of decalcification. The time for shaking may be 2-4 hours.

In one example embodiment, the decalcification is employed at room temperature.

### Decellularization

Decellularization refers to a process of removal of substantially all cellular components in the tissue. The resulting decellularized 3D-scaffold is substantially depleted of cellular cytoplasmic and nuclear material. Hereby, the decellularized 3D-scaffold may be described as substantially immunologically inert. Decellularization may be employed by physical means and/or decellularization media such as detergents, salts, and enzymes.

In one example embodiment, the decellularized 3D-scaffold has a concentration of DNA of less than 20 ng dsDNA/mg body part (dry weight).

In one example embodiment, the decellularized 3D-scaffold has a fragment length of the DNA of less than 200 bp.

According to one example embodiment, the step of decellularizing comprises trypsin and a chelating agent. Non-limiting examples of chelating agents are ethylenediaminetetraacetic acid (EDTA), nitrilotriacetic acid (NTA), iminodisuccinic acid (IDS), polyaspartic acid, S,S-ethylenediamine-N,N'-disuccinic acid (EDDS), methylglycinediacetic acid (MGDA), L-Glutamic acid N,N-diacetic acid, and tetrasodium salt (GLDA).

According to one example embodiment, the decellularization media comprises matrix metalloproteinases (MMP) and a chelating agent. Non-limiting examples of activated matrix metalloproteinases are MMP1, MMP2, MMP3, MMP7, MMP8, MMP9, MMP11, MMP13, and Membrane type 1-matrix metalloproteinase (MT1-MMP). The concentration of the MMP may be in the interval of 1-10 µg/ml and preferably 2-5 µg/ml.

Metalloproteinases are metalloproteinases that are calcium-dependent zinc-containing endopeptidases. Collectively, these enzymes are capable of degrading all kinds of extracellular matrix proteins, but also can process a number of bioactive molecules. They are known to be involved in the cleavage of cell surface receptors, the release of apoptotic ligands. MMPs are also thought to play a major role in cell behaviors such as cell proliferation, migration (adhesion/dispersion), differentiation, angiogenesis, apoptosis, and host defense.

In one example embodiment, the decellularization media comprises trypsin and ethylenediaminetetraacetic acid (EDTA). EDTA is often used with trypsin, an enzyme that acts as a protease to cleave the already existing bonds between integral proteins of neighboring cells within a tissue. Together, the EDTA-Trypsin combination make a good team for decellularizing decalcified tissues.

In one example embodiment, the step of decellularizing is performed in a temperature range of 15-37°C.

According to one example embodiment, the method further comprises a step of conditioning the decellularized 3D-scaffold. Conditioning may be employed by MMP-inhibitor such as hydroxymates and/or chelating agent such as carboxylates, thiols, and phosphinyls. Hydroxymates are particularly potent inhibitors of MMPs and other zinc-dependent enzymes, due to their bidentate chelation of the zinc atom.

According to one example embodiment, the method further comprises a step of freezing the decellularized 3D-scaffold of step c). Hereby, the decellularized 3D-scaffold is frozen and thawed before the step of recellularizing. The step of decellularization is preferably employed to a fresh body part, i.e. a body part that has not been frozen. The reason for that is that tissue, particularly tissue proteins, may be destroyed if frozen repeatedly.

### Stem cells

According to one example embodiment, the human stem cells are isolated from at least one of human bone marrow, blood, and adipose.

Stem cells isolated from blood is advantageous since it does not require any surgical operation to collect.

In one example embodiment said stem cells comprise mesenchymal stem cells (MSC). Mesenchymal stem cells (MSCs) also known as mesenchymal stromal cells or medicinal signaling cells are multipotent stromal cells that can differentiate into a variety of cell types, including osteoblasts (bone cells), chondrocytes (cartilage cells), myocytes (muscle cells) and adipocytes (fat cells which give rise to marrow adipose tissue).

In one example embodiment, said human stem cells comprise enriched stem cells such as CD271 and/or CD90 expressing cells. CD271 and CD90 are markers used to identify i.e. mesenchymal stem cells from diverse sources.

The official full name of CD271 is nerve growth factor receptor (TNFR superfamily, member 16). CD271, also called the LNGFR or p75 neurotrophin receptor, regulates neuronal growth, migration, differentiation, and cell death during stem cells. It is one of the two receptor types for the neurotrophins, a family of protein growth factors that stimulate neuronal cells to survive and differentiate. LNGFR is a member of the tumor necrosis factor receptor (TNF receptor) superfamily.

The official full name of CD90 is Thy-1 cell surface antigen. This gene encodes a cell surface glycoprotein and member of the immunoglobulin superfamily of proteins. The encoded protein is involved in cell adhesion and cell communication in numerous cell types, but particularly in cells of the immune and nervous systems. The encoded protein is widely used as a marker for hematopoietic stem cells.

According to one example embodiment, the human stem cells comprise unexpanded and undifferentiated human stem cells. The human stem cells may then differentiate in the recellularized 3D-scaffold to chondrocytes and/or osteocytes. Chondrocytes are the only cells found in healthy cartilage. They produce and maintain the cartilaginous matrix, which consists mainly of collagen and proteoglycans. An osteocyte is an oblate shaped type of bone cell with dendritic processes and is the most commonly found cell in mature bone tissue.

According to one example embodiment, a growth factor is inoculated together with the human stem cells into the decellularized 3D-scaffold

According to one example embodiment, the growth factor comprises chondrogenesis media and/or osteogenesis media.

According to one example embodiment, a growth factor is inoculated together with the human stem cells into said decellularized 3D-scaffold and wherein the growth factor comprises chondrogenesis media and/or osteogenesis media. Non-limiting examples of chondrogenesis medium and their concentrations include: MEM with alpha modification, 1% Penicillin & Streptomycin, 10% inactivated AB serum, 1% L-glutamine, TGF Beta 3 (10 ng/ml), Bone morphogenetic protein-2 (10 ng/ml), Bone morphogenetic protein-4 (10 ng/ml), Basic Fibroblast growth factor (10 ng/ml), Epidermal growth factor (1 ng/ml), Insulin-like growth factor (5 ug/ml), Transferrin (5 ug/ml), Sodium-Selenite (5 ug/ml), Proline (0.35 mM), Ascorbic acid-2-phosphate (0.17 mM) or L-Ascorbic acid (50 ug/ml), and Dexamethasone (100nM). Non-limiting examples of Osteogenesis medium and their concentrations include: Minimum essential medium (MEM) (High glucose), Penicillin & Streptomycin, 10% inactivated AB serum, 1% L-glutamine, L-Ascorbic acid (1 mM), Beta-Glycerophosphate (10 mM), and Dexamethasone (10 nM).

### Inoculation and Centrifugation

Cell attachment to a scaffold is a significant step toward successful tissue engineering. Cell inoculation is the first stage of cell attachment, and its efficiency and distribution can affect the final biological performance of the scaffold.

Efficiency in this context may be defined as seeding efficiency of at least 40% of seeded cells as determined by the quantity of cellular DNA within 5 hours after seeding.

Distribution in this context may be defined as percentage of area occupied by cells in tissue as enumerated and quantified by histology.

The cells may be inoculated by seeding, such as by injection or perfusion.

According to one example embodiment, the step of recellularizing further comprises centrifugating the inoculated 3D-scaffold. Centrifugation is advantageous since it may improve cell distribution within the body part. Centrifugation may also decrease the time for cell attachment. The centrifugation may be performed during at least 1 minute, at least 3 minutes or at least 5 minutes.

In one example embodiment at least 0.1 million cells/mm², at least 0.2 million cells/mm², at least 0.3 million cells /mm², at least 0.4 million cells/mm², or at least 0.5 million cells/mm² are seeded to the decellularized 3D-scaffold. In one example embodiment, 1 million cells/mm² are inoculated to the decellularized 3D-scaffold. The cells may be inoculated all at the same time or portion-wise in intervals.

The cells may be inoculated on top of the decellularized 3D-scaffold.

In one example embodiment, the inoculated decellularized 3D-scaffold is centrifugated directly after inoculation. In one example embodiment, the inoculated 3D-scaffold is centrifugated at least 5 min, at least 15 min, at least 30 min, at least 45 min or at least 60 min after seeding.

### Culturing

In one example embodiment, the step of recellularizing further comprises culturing of cells in a bioreactor for 1-8 weeks, and preferably 2-4 weeks. A result of this is that the cultured cells express human genes.

### Product

According to a second aspect of the disclosure, a tissue-engineered human graft as herein described is provided.

According to one example embodiment, the tissue-engineered human graft as herein described comprises soft bone, cartilage, and/or skin. Further examples of tissue-engineered human grafts obtainable by the invention are skin and blood vessels.

According to one example embodiment, a tissue engineered human graft for use in therapy is provided.

According to one example embodiment, a tissue engineered human graft for treatment of Osteoarthritis is provided.

According to one example embodiment, a tissue engineered human graft for treatment of joint diseases is provided. Non-limiting examples of joint diseases are Osteoarthritis, Post-traumatic arthritis, Rheumatoid arthritis, Ankylosing spondylitis, Osteoporosis, Discoid meniscus, Bone disorders such as Bone Cancer, Bone Density, Bone Infections, Osteogenesis Imperfecta, Osteonecrosis, Osteoporosis, Paget's Disease of Bone, and Rickets.

According to one example embodiment, a tissue engineered human graft as herein described for use as ink for 3D printing.

### Definitions

Generally, all terms used in the claims are to be interpreted according to their ordinary meaning in the technical field, unless explicitly defined otherwise herein. All references to "a/an/the [element, device, component, means, step, etc.]" are to be interpreted openly as referring to at least one instance of the element, device, component, means, step, etc., unless explicitly stated otherwise. The steps of any method disclosed herein do not have to be performed in the exact order disclosed, unless explicitly stated.

Decellularization refers to the process of removal of substantially all cellular components in a tissues/organ by physical, chemical and/or enzymatic methods.

Recellularization refers to the process of delivering cells to a decellularized 3D-scaffold and culturing the cells such that the cells proliferate and differentiate to generate a personalized tissue-engineered human graft.

Decalcification describes the technique for removing calcium ions and/or other mineral from bone or other calcified tissues to soften.

Autologous stands for obtained from the same individual.

Inoculation refers to the spread of cells to a tissue or body part for cell culture activities.

### BRIEF DESCRIPTION OF THE DRAWINGS

The disclosure will be described in more detail with reference to the appended schematic drawings, which show an example of a presently preferred embodiment of the disclosure.
Fig. 1 illustrates a flow chart for a method for generation of personalized tissue-engineered human grafts for direct use according to at least one example embodiment of the inventive concept;
Fig. 2a illustrates a flow chart for a method for recellularization of a decellularized 3D-scaffold according to at least one example embodiment of the inventive concept;
Fig. 2b illustrates a flow chart for a method for recellularization of a decellularized 3D-scaffold;
Fig. 3a and 3b illustrate a comparison of an inoculated 3D-scaffold without centrifugation and an inoculated 3D-scaffold with centrifugation.

### DETAILED DESCRIPTION

The present disclosure will now be described more fully hereinafter with reference to the accompanying drawings, in which currently preferred embodiments of the disclosure are shown. The present disclosure may, however, be embodied in many different forms and should not be construed as limited to the embodiments set forth herein; rather, these embodiments are provided for thoroughness and completeness, and to fully convey the scope of the disclosure to the skilled addressee. Like reference characters refer to like elements throughout.

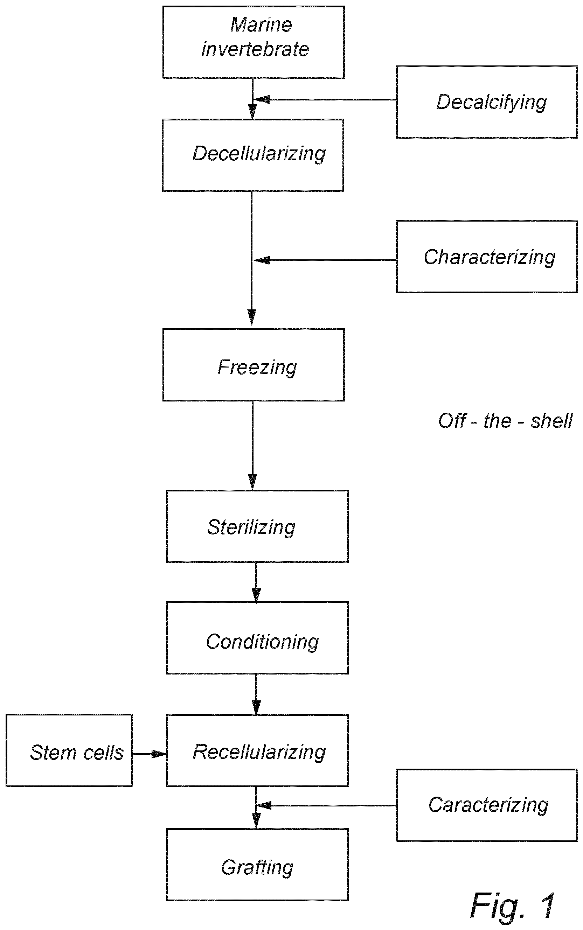

Fig. 1 illustrates a flow chart for a method for generation of personalized tissue-engineered human grafts for direct use. First, a body part taken from marine invertebrates is collected and washed with distilled water to remove debris. Possibly the body part is maintained in flowing sea water until needed.

In cases of bony or hard structures, the body part is preferably decalcified in order to make it softer. Non-limiting examples of decalcification media are hydrochloric acid, nitric acid, formic acid, and ethylenediaminetetraacetic acid (EDTA). Optionally, the body part is subjected to stirring during decalcifying. The time for decalcification may be two hours and the temperature may be room temperature. Subsequently the decalcified body part is preferably repeatedly washed with distilled water.

Next, the body part is decellularized into a decellularized 3D-scaffold which means substantially all cells and DNA are removed. Decellularization is preferably also performed during stirring. The time for decellularization may be two hours and the temperature may be 37 °C. Non-limiting examples of decellularization media are trypsin-EDTA. The outcome of decalcification and decellularization processes may be analyzed by histology.

The decellularized 3D-scaffold may now be frozen until later use. Hereby, an off-the-shell product is produced.

Before the decellularized 3D-scaffold is recellularized, it is preferably is sterilized and conditioned. Sterilization may be performed by subjecting the 3D-scaffold to peracetic acid in sterile phosphate-buffered saline (PBS) for 1 hour on a shaker at room temperature. Bony tissue may be washed with sterile PBS three times, each time for 20 minutes.

Recellularization is performed by inoculating, such as seeding, the decellularized 3D-scaffold with human stem cells. Preferably the stem cells are suspended in liquid medium and seeded onto a piece of the decellularized 3D-scaffold, e.g. by use of pipette. The stem cells are then incubated and/or centrifuged for some time to allow cell attachment. The stem cells may originate from e.g. bone marrow, adipose, or blood. The stem cells are subsequently allowed to expand and differentiate in the recellularized 3D-scaffold. Hereby, they need not be expanded and differentiated prior to inoculation. The stem cells may be cultured in a bioreactor, e.g. for 4 weeks. Growth factors for this include osteogenesis media and/or chondrogenesis media. The osteogenesis medium may be supplemented with heat inactivated human AB serum, L-glutamine, antimycotic-antibiotic, L-Ascorbic acid, β Glycerophosphate, and/or dexamethasone. The chondrogenesis medium may be supplemented with heat inactivated human AB serum, L-glutamine, anti-mycotic-antibiotic, bone morphogenetic protein 2 (BMP-2), basic fibroblast growth factor (bFGF), epidermal growth factor (EGF), insulin-transferrin-selenium (ITS), and/or dexamethasone.

In order to control the outcome of the recellularization the recellularized tissue may be characterized by methods known in the art such as histology, immunohistochemistry, and/or by gene expression.

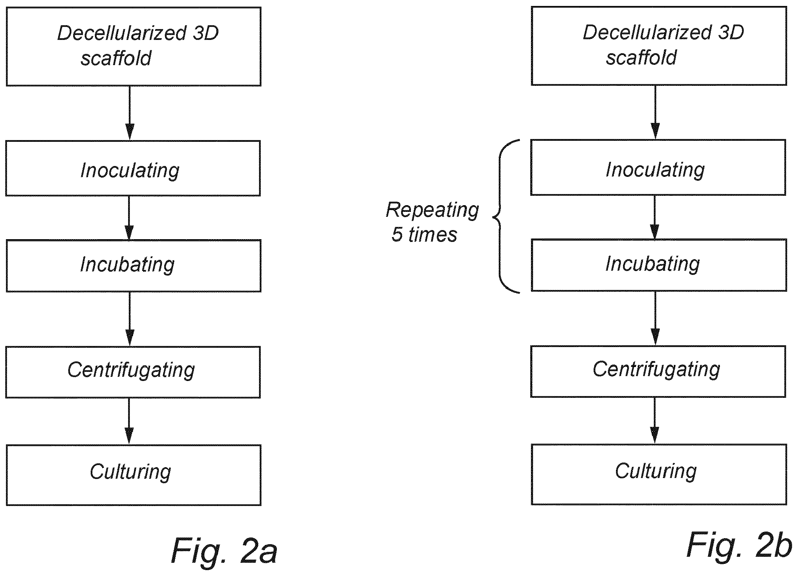

Fig. 2a and 2b illustrate a flow chart for a method for recellularizing a decellularized 3D-scaffold. In Fig. 2a all human stem cells are inoculated altogether in one portion. The number of human stem cells may for example be 1 million. The human stem cells may be inoculated on top of the body part. After inoculation the inoculated 3D-scaffold is incubated for 60 min. Then the incubated 3D-scaffold if centrifuged and cultured, for 5 min and cultured for 4 weeks.

Fig. 2b illustrates a method recellularization wherein the human stem cells are inoculated sequentially. The number of human stem cells may be for example 0.2 million and the human stem cells may be inoculated on top. After the inoculation, the inoculated 3D-scaffold is left for 5 min. Then another portion of 0.2 million cells are inoculated. The procedure is repeated for five times. The inoculated 3D-scaffold is then centrifuged e.g. for 1 min and thereafter cultured for 4 weeks.

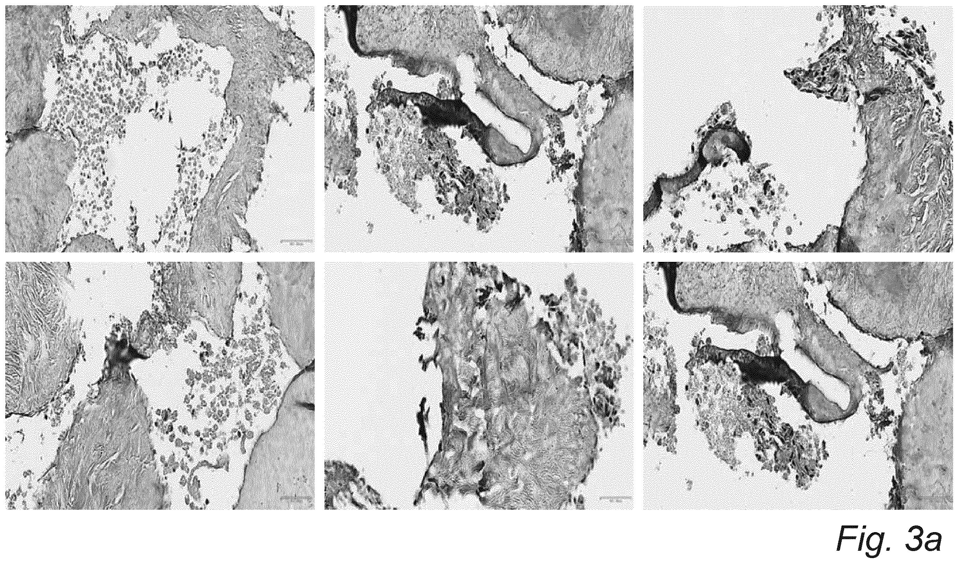

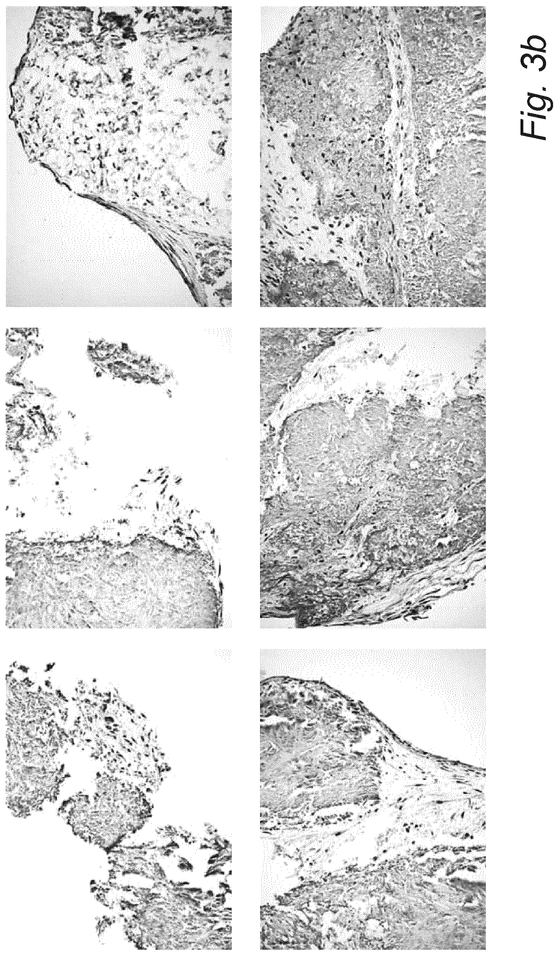

Fig. 3 illustrates a comparison of an inoculated 3D-scaffold that has been centrifugated and an inoculated 3D-scaffold without centrifugation. Many more human stem cells have been inoculated deeper into the 3D-scaffold with centrifugation compared to no centrifugation.

The skilled person realizes that a number of modifications of the embodiments described herein are possible without departing from the scope of the disclosure, which is defined in the appended claims.

### Examples

### Materials and methods

Viable starfishes (n=10) kept in aquaculture water were transported to lab. All arms were removed from an individual initially possessing five arms and were cleaned thoroughly with distilled water.

### Decalcification and decellularization of starfish bony tissue

The tissue (5x0.8x0.2 cm) was decalcified in 1N hydrochloric acid on a shaker (rpm= 240) for 2 hours at room temperature and washed with distilled water for three times (3x20 min) under the same shaking condition. After washing, the tissue was decellularized with 0.05% of trypsin-EDTA on a shaker (rpm= 70) for 2 hours at 37°C. Bony tissue was washed with distilled water for three times (3x20 min) with the same rpm on shaker at room temperature. Decellularized bony tissue was then sterilized. Decellularized starfish bony tissue pieces were sterilized in 0.1 % of peracetic acid in sterile PBS for 1 hour on a shaker (rpm= 240) at room temperature. Bony tissue was washed with sterile PBS three times, each time for 20 minutes.

### Characterization of decellularized starfish bony tissue

After decalcification and decellularization, tissue biopsies were collected and processed for histology. The biopsies were fixed in 4% formaldehyde for 24 hours and dehydrated by standard tissue processing and paraffin embedding. Sections of 5 µm thickness were cut using microtome. After series steps of rehydration hematoxylin and eosin (HE) staining were performed. Decalcified slides were as a control and 4', 6-diamino-2 phenylindole staining were also performed to confirm the HE findings.

### Conditioning of decellularized bone tissue

Sterilized tissue pieces were cut into 0.4x0.4x0.1cm in a Petri dish and placed in 6 wells of a 24-well transwell plate. Sterilized tissue was placed in DMEM, supplemented with 10% heat inactivated human AB serum, 1% L-glutamine, 1% antimitotic-antibiotic over night at 37°C incubator.

### Recellularization of starfish bone - Seeding of decellularized starfish bony tissue with BM-MSC

The washed tissue pieces were placed on 12-well transwell membrane (0.4 µm pore size) and the MSC isolated human bone marrow, were seeded at approximately 30x106 cells per piece. The cells were suspended in 200 µl medium and seeded onto the tissue piece using 10 µl pipette tip and then incubated for an hour to allow cell attachment or centrifuged at 1000 rpm for 5 min. Later, 2 ml medium was added gently along the sides of the well and cultured for 4 weeks in transwell plates in a bioreactor. One set of transwells were cultured in osteogenesis medium, while cells in the second set of transwells were cultured in chondrogenesis medium.

The osteogenesis medium was supplemented with 10% heat inactivated human AB serum, 1% L-glutamine, 1%, antimycotic-antibiotic, 1 mM L-Ascorbic acid, 10mM β Glycerophosphate, 10nM Dexamethasone. The chondrogenesis medium (MEM) was supplemented with 10% heat inactivated human AB serum, 1% L-glutamine, 1% anti-mycotic-antibiotic, BMP-2, basic fibroblast growth factor (bFGF), epidermal growth factor (EGF), insulin-transferrin-selenium (ITS), and dexamethasone.

### Characterization of recellularized 3D-scaffold

### 1) Histology

By end of recellularization biopsies from each chamber were collected and fixed in 4% formaldehyde for 24 hours. Samples were dehydrated with ascending concentrations of ethanol, and Xtrasolv by tissue processor and embedded in paraffin. The paraffin blocks were sectioned at 5um thickness and stained with hematoxylin and eosin (HE). Simultaneously 4', 6-diamino-2 phenylindole (DAPI) staining were also performed to conform the HE findings.

### 2) Immunohistochemistry

Recellularized tissue were performed immunohistochemistry staining. The slides were rehydrated, antigen retrieved and endogenous peroxidase blocked by standard procedure. Primary antibodies were used for staining included Osteocalcin (1:50MA), Collagen I (1:100), DMP-1 (1:100), RANK (1:200), CD105 (1:100), CD 68 (1:100). The sections were incubated with primary antibodies overnight at 4°C. Sections were washed with PBS and incubated with secondary antibody (Invitrogen) for 10 min at room temperature. Colorimetric identification was performed using 3, 3'-diaminobenzidine and counter-stained with hematoxylin. Decellularized sections were as reference and sections which were absent primary antibody on the same slides were as negative controls.

### 3) Detection of human genes in the cultured 3D-scaffold

RNA from cells cultured in chondrogenesis medium, recellularized marine tissue and a negative control (water) were extracted using Qiagen RNeasy Mini Kit.

Reverse transcription: The 10 extracted samples were reverse transcribed using TATAA GrandScript cDNA Synthesis Kit. qPCR: The preamplified samples were thawed by adding RNase free water to each sample and mixed. The diluted samples and no-template control (water) were analyzed in duplicates for 20 genes, using same assays as in preamplification, in a Bio-Rad cfx384 Real-Time System and then quantified according to standard methods.

## Claims

1. A method for generation of personalized tissue-engineered human grafts, said method comprising the following steps in the order named:
a) providing a body part taken from a marine invertebrate, said body part comprising at least two biologically interconnected tissues selected from the group consisting of connective tissue, muscle tissue, epithelia tissue, and nervous system tissue;
b) washing said body part with distilled water;
c) decellularizing said washed body part to remove cells and DNA, to provide a decellularized 3D-scaffold with extracellular matrix (ECM) proteins, and, optionally,
d) recellularizing said decellularized 3D-scaffold by:
inoculating human stem cells into said decellularized 3D-scaffold, and
in vitro culturing said human stem cells in said inoculated 3D-scaffold.

2. The method according to claim 1, wherein said human stem cells are isolated from at least one of bone marrow, blood, and adipose.

3. The method according to claim 1 or 2, wherein said human stem cells comprise enriched human stem cells such as CD271 and/or CD90 expressing cells.

4. The method according to any one of claims 1-3, wherein said step of recellularizing further comprises centrifugating said inoculated 3D-scaffold.

5. The method according to any one of claims 1-4, wherein said human stem cells in said inoculated 3D-scaffold is cultured in a bioreactor for 1-8 weeks, and preferably 2-4 weeks.

6. The method according to any one of claims 1-5, a growth factor is inoculated together with the human stem cells into said decellularized 3D-scaffold and wherein said growth factor comprises chondrogenesis media and/or osteogenesis media.

7. The method according to any one of claims 1-6, further comprising a step of decalcifying said washed body part of step b) before said step of decellularizing.

8. The method according to any one of claims 1-8, said step of decellularizing comprises trypsin and a chelating agent.

9. The method according to any one of claims 1-7, said decellularization media comprises activated matrix metalloproteinases (MMP) and a chelating agent.

10. The method according to any one of claims 1-9, wherein said body part derive from Echinoderms, Poriferans, Cnidarians, Mollusks, or Arthropods; or wherein said body part derive from Echinoderms such as starfish, sea urchins, sand dollars, sea cucumbers, or sea lilies.

11. The method according to any one of claims 1-10, further comprising a step of freezing said decellularized 3D-scaffold of step c).

12. A tissue-engineered human graft obtained by the method according to claim 1.

13. The tissue engineered human graft according to claim 12, wherein said graft comprises soft bone and/or cartilage.

14. The tissue engineered human graft according to claim 12 or 13, for use in therapy.

15. The tissue engineered human graft according to any one of claims 12-14, for use as ink for 3D printing.
